# EUROPÄISCHE PATENTANMELDUNG

(11) **EP 0 673 917 A1**
(43) Veröffentlichungstag der Anmeldung: **27.09.1995**
(21) Anmeldenummer: 95103936.1
(22) Anmeldetag: 17.03.1995
(51) Int. Cl.: C07C 209/10, C07C 211/48

(54) **Verfahren zur Herstellung von Arylbenzylaminen**

(30) Priorität: 25.03.1994 DE 4410360
(71) Anmelder: BASF AKTIENGESELLSCHAFT, D-67056 Ludwigshafen (DE)
(72) Erfinder: Hammer, Karl-Heinz, D-67127 Rödersheim-Gronau (DE); Husslein, Gerd, Dr., D-67098 Bad Dürkheim (DE)

(57) **Zusammenfassung**

Verfahren zur Herstellung von Arylbenzylaminen durch Umsetzung von Arylaminen mit einem Benzylchlorid, dadurch gekennzeichnet, daß die Umsetzung bei gleichzeitiger Anwesenheit eines Phasentransferkatalysators und einer Base aus der Gruppe der anorganischen Basen und der Salze schwacher organischer Säuren durchgeführt wird.

## Beschreibung

Die Erfindung betrifft ein verbessertes Verfahren zur Herstellung von Arylbenzylaminen durch Umsetzung von Arylaminen mit Benzylchlorid bei gleichzeitiger Gegenwart einer Base und eines Phasentransferkatalysators.

Für die Herstellung von Arylbenzylaminen sind mehrere Verfahren bekannt. Arylbenzylamine sind beispielsweise durch Kondensation von Arylaminen mit Benzylchlorid in Gegenwart einer Base wie Magnesiumoxid erhältlich (vgl. PL 57214).

Desweiteren können Arylbenzylamine durch Reaktion von Arylaminen mit Benzylalkoholen in Gegenwart von Triphenylphosphit erhalten werden (vgl. DE-A-26 06 363).

Eine weitere Möglichkeit zur Herstellung von Arylbenzylaminen besteht in der reduktiven Alkylierung von Benzylaminen oder Benzylschiffbasen (vgl. Trapani et al., Synthesis, Vol. 12 (1983), Seiten 1013 ff.; Ayyangar et al., J.Chem.Technol. Biotechnol. Vol. 51 (1991), Heft 3, Seiten 293 ff.).

Ein Nachteil der bekannten Verfahren ist, daß das nach der chemischen Umsetzung vorliegende Reaktionsgemisch das gewünschte Arylbenzylamin in einer zur direkten Weiterverwendung für die meisten Fälle ungenügenden Reinheit enthält, so daß weitere Aufarbeitungsschritte erforderlich sind. So fällt beispielsweise N-Benzyl-N-ethylanilin nach den obigen Verfahren im Gemisch mit Lösungsmitteln, unumgesetzten Ausgangsstoffen und Nebenprodukten an und muß vor der Weiterverwendung destilliert werden.

Aufgabe der vorliegenden Erfindung war es daher, ein Verfahren bereitzustellen, das bei guten Raum-Zeit-Ausbeuten Arylbenzylamine von hoher Reinheit ergibt.

Demgemäß wurde ein Verfahren zur Herstellung von Arylbenzylaminen durch Umsetzung von Arylaminen mit einem Benzylchlorid gefunden, bei dem die Umsetzung bei gleichzeitiger Gegenwart eines Phasentransferkatalysators und einer Base aus der Gruppe der anorganischen Basen und der Salze schwacher organischer Säuren durchgeführt wird.

Als Arylamine eignen sich erfindungsgemäß primäre oder sekundäre Arylamine, wobei der oder die Arylreste gegebenenfalls substituiert sein können.

In einer bevorzugten Ausführungsform des erfindungsgemäßen Verfahrens wird als Arylamin ein im Arylrest gegebenenfalls substitutiertes N-Alkylarylamin eingesetzt.

Beispiele für geeignete N-Alkylarylamine sind N-Ethylanilin, N-Methylanilin, N-Ethyl-o-toluidin, N-Ethyl-4-chloranilin, 4-Methoxianilin und 4-Acetylaminoanilin. Erfindungsgemäß besonders bevorzugte N-Alkylarylamine sind N-Methylanilin und N-Ethylanilin.

Die Arylamine können einzeln oder in Mischung eingesetzt werden. Zweckmäßigerweise werden im erfindungsgemäßen Verfahren einzelne Arylamine eingesetzt.

Die im erfindungsgemäßen Verfahren eingesetzten Benzylchloride sind das unsubstituierte Benzylchlorid oder im Phenylring substituierte Benzylchloride. Beispiele für geeignete substituierte Benzylchloride sind o- und p-Chlorbenzylchlorid, p-Nitrobenzylchlorid und o-Methylbenzylchlorid. Vorzugsweise wird das unsubstituierte Benzylchlorid verwendet.

Die im erfindungsgemäßen Verfahren eingesetzten Ausgangsstoffe Benzylchlorid und Arylamine werden in der Regel in stöchiometrischem Verhältnis oder mit einem bis zu 5 mol%-igen Benzyl-chlorid-Überschuß eingesetzt.

Erfindungsgemäß geeignete Basen aus der Gruppe der anorganischen Basen und der Salze schwacher organischer Säuren (im folgenden zusammenfassend auch als erfindungsgemäße Basen bezeichnet) sind z.B. Hydroxide, basische Oxide, Hydrogenphosphate, Acetate, Carbonate und Hydrogencarbonate von Metallen. Vorzugsweise werden anorganischen Basen verwendet. Bevorzugt sind hierbei Alkali- und/oder Erdalkalicarbonate oder -hydrogencarbonate. Besonders bevorzugt sind hiervon Alkalicarbonate, insbesondere Natriumcarbonat.

Die erfindungsgemäßen Basen werden in der Regel stöchiometrisch oder im molaren Überschuß eingesetzt. Vorzugsweise wird die Base in einer Menge von 1 bis 2 mol pro mol Arylamin, besonders bevorzugt von 1,01 bis 1,1 mol pro mol Arylamin eingesetzt. Die Basen werden im allgemeinen als wäßrige Lösung oder als Feststoff eingesetzt. Vorteilhaft werden die erfindungsgemäßen Basen als wäßrige Lösungen verwendet, um Anbackungen an Apparateteilen und Materialabrasion zu vermeiden.

Als Phasentransferkatalysator können die hierfür üblicherweise bekannten verwendet werden. Beispiele für besonders geeignete Phasentransferkatalysatoren sind Tetrabutylammoniumchlorid bzw. -bromid, Dibenzyl-dimethylammoniumchlorid, Benzyl-trimethyl-ammoniumchlorid, Benzyl-dimethyl-C₁₂₋₁₄-alkylammoniumchlorid. Bevorzugt ist hiervon die Verwendung von Benzyl-dimethyl-C₁₂₋₁₄-alkylammoniumchlorid. Die in reiner Form meist festen Phasentransfer-Katalysatoren können als solche oder vorzugsweise in gelöster Form eingesetzt werden.

In einer besonders bevorzugten Ausführungsform des erfindungsgemäßen Verfahrens enthält die Reaktionsmischung mindestens ein tertiäres Amin und der Phasentransfer-Katalysator wird in situ durch die Reaktion des tertiären Amins mit dem ggf. substituierten Benzylchlorid erzeugt.

Die Vorstufe des Phasentransferkatalysators kann so als tertiäres Amin in flüssiger Form zur Reaktionsmischung gegeben werden, das mit Benzylchlorid zur wirksamen Quartärverbindung reagiert.

Hierfür werden vorzugsweise tertiäre aliphatische Amine wie z.B. Triethylamin, Tripropylamin, Tributylamin, Dimethyldodecylamin, Dimethyl-C₁₂₋₁₄-alkylamin ("Dimethylpalmkernfettamin") verwendet.

Der Phasentransfer-Katalysator wird im allgemeinen in einer Menge, bezogen auf Arylamin, von 0,5 bis 5 Gew.-%, vorzugsweise 1 bis 2 Gew.-% eingesetzt.

In einer besonders bevorzugten Ausführungsform des erfindungsgemäßen Verfahrens wird N-Ethylanilin mit Benzylchlorid zu Ethylphenylbenzylamin umgesetzt, wobei als Phasentransferkatalysatorvorstufe N,N-Dimethyl-dodecylamin und als anorganische Base Natriumcarbonat eingesetzt wird.

Das erfindungsgemäße Verfahren wird vorteilhaft ohne zusätzliches organisches Lösungsmittel durchgeführt. Gegebenenfalls kommen aber auch unter den Reaktionsbedingungen inerte, organische Lösungsmittel, vorzugsweise mit Wasser nicht oder wenig mischbare Lösungsmittel wie z.B. Toluol oder 1,2-Dichlorethan in Betracht. Vorzugsweise werden daher beim erfindungsgemäßen Verfahren nur die erfindungsgemäßen Arylamine, Benzylchloride sowie die ggf. als wäßrige Lösung vorliegende erfindungsgemäße Base und der Phasentransferkatalysator bzw. die zu seiner in situ-Erzeugung verwendeten tertiären Amine eingesetzt.

Das erfindungsgemäße Verfahren kann mit oder ohne Wasser als Verdünnungsmittel durchgeführt werden.

Beim erfindungsgemäßen Verfahren kann bei konstanter Temperatur oder unter Anwendung eines Temperaturprofils gearbeitet werden. Hierbei wird in der Regel bei erhöhten Temperaturen, vorzugsweise im Temperaturbereich von 80 bis 110°C und besonders bevorzugt von 90 bis 100°C gearbeitet.

Bei Durchführung des erfindungsgemäßen Verfahrens im Temperaturbereich von 80 bis 110°C bleibt die helle Farbe des produzierten rohen Arylbenzylamins beim Lagern auch ohne besondere Vorkehrungen wie die Anwendung einer Stickstoffabdeckung erhalten.

Das erfindungsgemäße Verfahren kann drucklos oder unter Druck durchgeführt werden.

Das erfindungsgemäße Verfahren kann beispielsweise wie folgt durchgeführt werden:
Die erfindungsgemäße Base, z.B. eine anorganische Base, wird in einer Vorlage von Wasser in Lösung gebracht. Anschließend gibt man das Arylamin und den Katalysator (vorzugsweise in Form der Vorstufe tertiäres Amin) zu, erwärmt und läßt bei ca. 80°C das Benzylchlorid so zulaufen, daß sich die Reaktionsmischung durch die schwach exotherme Umsetzung auf 94 bis 96°C erwärmt und bei dieser Temperatur gehalten werden kann. Man führt die Reaktion bei ca. 95°C bis zur vollständigen Umsetzung weiter, setzt gegebenenfalls zur Auflösung der anorganischen Bestandteile und zur Abkühlung weiteres kaltes Wasser zu und trennt anschließend bei ca. 30°C die das gewünschte Benzylamin enthaltende organische Phase in üblicher Weise ab.

Die nach dem Verfahren der Erfindung herstellbaren Arylbenzylamine sind wertvolle Ausgangsstoffe für Farbstoffe und Hilfsmittel. So kann das wie oben hergestellte rohe N-Benzyl-N-ethylanilin z.B. direkt zur Sulfosäure umgesetzt werden, die in Lebensmittel- und Gebrauchsfarbstoffe (z.B. Triphenylmethanfarbstoffe für den Sanitärbereich) eingeht.

Das erfindungsgemäße Verfahren hat zahlreiche Vorteile. Im Vergleich zu den bekannten Verfahren liefert das erfindungsgemäße Verfahren auf einfache und wirtschaftliche Weise Arylbenzylamine in hoher Ausbeute und Reinheit.

Die hierzu notwendigen technischen Anlagen sind einfach und können sich auf einen Rührapparat und einen Abluftkühler beschränken, wobei das bei den bekannten Verfahren bei hohen Temperaturen vorhandene Materialproblem nicht auftritt.

Eine Überwachung oder Regelung des pH-Wertes der Reaktion ist im allgemeinen nicht erforderlich. Die gute Qualität der erzeugten rohen Arylbenzylamine wird zuverlässig und ohne erhöhten Überwachungsaufwand erreicht, da auch bei Abweichung von den vorgesehenen Einsatzstoffverhältnissen die Bildung der zu erwartenden Nebenprodukte (Benzylalkohole, Dibenzylether) nahezu vollständig unterdrückt wird. Fehldosierungen bei den eingesetzten Basen sowie dem Arylamin und Benzylchlorid oder Abweichungen von den vorgegebenen Prozeßparametern können daher jederzeit ohne Qualitätseinbuße korrigiert werden.

### Beispiele

### Beispiel 1

In einem Rührapparat aus Stahl/Email wurden 1750 Gew.-Teile Wasser vorgelegt. Unter Rühren wurden hierzu 750 Gew.-Teile Natriumcarbonat gegeben. Anschließend wurden 1400 Gew.-Teile N-Ethylanilin und 38 Gew.-Teile N,N-Dimethyl-C₁₂₋₁₄-alkylamin hinzugefügt und die Mischung unter Rühren auf 80°C aufgeheizt. Danach wurden 1496 Gew.-Teile Benzylchlorid (Zulaufgeschwindigkeit: 700 Gew.-Teile pro Stunde) zugeführt. Die Reaktionsmischung erwärmte sich hierbei und wurde durch Kühlung auf einer Temperatur von 94 bis 96°C gehalten. Nach beendeter Zugabe wurde noch 12 Stunden bei dieser Temperatur gerührt. Man ließ anschließend 500 Gew.-Teile Wasser zulaufen und die Reaktionsmischung auf 30°C abkühlen. Der Rührer wurde dann abgestellt und die Mischung für 1 Stunde zur Phasentrennung stehen gelassen. Die untere wäßrige Phase wurde der Wiederaufbereitung zugeführt. Die obere hellgelbe organische Phase bestand aus 96 Gew.-% N-Benzyl-N-ethylanilin, 0,5 Gew.-% Benzylchlorid, 0,5 Gew.-% N-Ethylanilin, 2 Gew.-% Benzylalkohol und 1 Gew.-% Dibenzylether. Die organische Phase konnte ohne weitere Aufarbeitung weiter verwendet werden, z.B. zur Sulfonierung zu N-Benzyl-N-ethylanilinsulfosäure.

### Beispiel 2

In einem Rührapparat aus Stahl/Email wurden 1400 Gew.-Teile N-Ethylanilin und 38 Gew.-Teile N,N-Dimethyl-C₁₂₋₁₄-alkylamin vorgelegt. Unter Rühren wurden 750 Gew.-Teile Natriumcarbonat eingetragen. Anschließend wurde auf 90°C aufgeheizt und 1496 Gew.-Teile Benzylchlorid genügend langsam zugegeben, daß die Temperatur bei ca. 95°C blieb. Es wurde anschließend 10 Stunden bei 95°C und 14 Stunden bei 110°C gerührt. Danach wurde das Reaktionsgemisch auf eine Vorlage von 2500 Gew.-Teilen Wasser abgelassen und die Phasen wie unter Beispiel 1 beschrieben getrennt. Das Reaktionsgemisch bestand zu über 99 Gew.-% aus N-Benzyl-N-ethylanilin.

### Beispiel 3 (Vergleich)

In eine Vorlage von 1380 Gew.-Teilen N-Methylanilin wurden 1620 Gew.-Teile Benzylchlorid und 99 Gew.-Teile Natriumcarbonat eingetragen. Anschließend wurde 4 Stunden bei 105-110°C nachgerührt und auf 40°C abgekühlt. Nach der Zugabe von 5000 Gew.-Teilen Wasser wurden die gebildeten Phasen getrennt und die organische Produktphase mit 130 Gew.-Teilen Aktivkohle und 2560 Gew.-Teilen Wasser gerührt. Nach dem Abfiltrieren der festen Bestandteile wurde mit Wasser auf 15000 Gew.-Teile aufgefüllt, nachgerührt und die untere organische Phase abgetrennt. Es wurden 2250 Gew.-Teile eines Produktes der Zusammensetzung 87,1 Gew.-% N-Methyl-N-benzylanilin, 6,2 Gew.-% Benzylchlorid, 4,8 Gew.-% N-Methylanilin und 0,3 Gew.-% Benzylalkohol erhalten.

### Beispiel 4

Zu einer Vorlage von 750 Gew.-Teilen Natriumcarbonat in 1750 Gew.-Teilen Wasser wurden 1237 Gew.-Teile N-Methylanilin und 30 Gew.-Teile Dimethyl-C₁₂₋₁₄-amin gegeben. Nach dem Aufheizen auf 80°C wurden in 2 Stunden 1496 Teile Benzylchlorid so zudosiert, daß die Temperatur der Reaktionsmischung auf 94 bis 96°C anstieg und sich während des weiteren Zulaufs nicht weiter erhöhte. Nach 12 Stunden Nachrühren bei 95°C wurde auf 50°C abgekühlt, mit 500 Gew.-Teilen Wasser versetzt, unter Rühren auf 30 bis 35°C abgekühlt und die organische Phase abgetrennt. Es wurden 2250 Gew.-Teile eines Produktes der Zusammensetzung 97,5 Gew.-% N-Methyl-N-benzylanilin, 0,6 Gew.-% Benzylchlorid, 0,8 Gew.-% N-Methylanilin und 0,3 % Benzylalkohol erhalten.

### Beispiel 5

In eine Vorlage von 1237 Gew.-Teilen N-Methylanilin wurden bei Umgebungstemperatur unter Rühren 750 Gew.-Teile Natriumcarbonat und 30 Gew.-Teile Dimethyl-C₁₂₋₁₄-amin eingetragen. Nach dem Aufheizen auf 90°C wurden in 2 Stunden 1496 Gew.-Teile Benzylchlorid so zudosiert, daß die Temperatur der Reaktionsmischung auf 94 bis 96°C anstieg und sich während des weiteren Zulaufs nicht weiter erhöhte. Anschließend wurde 5 Stunden bei 95°C und weitere 20 Stunden bei 105 bis 110°C nachgerührt. Durch Zugabe von 2500 Gew.-Teilen Kaltwasser wurde unter Rühren auf 50°C abgekühlt und nach einer 30-minütigen Ruhezeit die organische Phase abgetrennt. Es wurden 2270 Gew.-Teile eines Produktes der Zusammensetzung 99,1 Gew.-% N-Methyl-N-benzylanilin, 0,2 Gew.-% Benzylchlorid, 0,15 Gew.-% N-Methylanilin und 0,3 Gew.-% Benzylalkohol erhalten.

## Patentansprüche

1. Verfahren zur Herstellung von Arylbenzylaminen durch Umsetzung von Arylaminen mit einem Benzylchlorid, dadurch gekennzeichnet, daß die Umsetzung bei gleichzeitiger Anwesenheit eines Phasentransferkatalysators und einer Base aus der Gruppe der anorganischen Basen und der Salze schwacher organischer Säuren durchgeführt wird.

2. Verfahren nach Anspruch 1, dadurch gekennzeichnet, daß als anorganische Base Alkali- und/oder Erdalkalicarbonate oder -hydrogencarbonate verwendet werden.

3. Verfahren nach Anspruch 2, dadurch gekennzeichnet, daß als anorganische Base Alkalicarbonate eingesetzt werden.

4. Verfahren nach einem der Ansprüche 1 bis 3, dadurch gekennzeichnet, daß als Arylamin ein gegebenenfalls im Phenylring substituiertes N-Alkylanilin verwendet wird.

5. Verfahren nach einem der Ansprüche 1 bis 4, dadurch gekennzeichnet, daß die Reaktionsmischung mindestens ein tertiäres Amin enthält und der Phasentransferkatalysator in situ durch Reaktion des tertiären Amins mit Benzylchlorid hergestellt wird.
